# EUROPÄISCHE PATENTSCHRIFT

(11) **EP 1 369 036 B1**
(45) Veröffentlichungstag und Bekanntmachung des Hinweises auf die Patenterteilung: **24.05.2006**
(21) Anmeldenummer: 03012814.4
(22) Anmeldetag: 05.06.2003
(51) Int. Cl.: A01L 11/00, A01L 15/00

(54) **Verfahren zur Ermittlung der Druckkraftverhältnisse im Huf, insbesondere von Equiden**
Method for determining the compression force ratios in a hoof, in particular for horses
Procédé pour déterminer les rapports des forces de pression dans un sabot, en particulier un sabot de cheval

(30) Priorität: 05.06.2002 DE 20208742 U
(43) Veröffentlichungstag der Anmeldung: 10.12.2003
(73) Patentinhaber: Ritzinger, Robert, Dipl.-Ing. (FH), 83556 Griesstätt (DE)
(72) Erfinder: Ritzinger, Robert, Dipl.-Ing. (FH), 83556 Griesstätt (DE)
(74) Vertreter: Kandlbinder, Markus Christian

(56) Entgegenhaltungen:
- DE-A- 19 707 413
- FR-A- 2 801 490
- US-A- 6 038 935

## Beschreibung

Die Erfindung betrifft ein Verfahren zur Ermittlung der Kraftverhältnisse an bzw. in Hufen, insbesondere von Equiden, gemäß dem Oberbegriff des Anspruchs 1.

Für eine aussagekräftige Bewegungsanalyse und / oder Lahmheitsdiagnostik bei Huftieren, aber auch bei Beurteilungen von Fehlstellungen der Gliedmaßen, insbesondere bei Pferden, sind neben dem subjektiven (Bild-) Wahmehmungsvermögen des Menschen verstärkt objektive Messdaten erforderlich. Die steigende Bedeutung der objektiven Erfassung des Bewegungsablaufes spiegelt sich nicht nur in der Zahl der getätigten Schutzrechtsanmeldungen wieder, sondern auch in den vielfältigen Studien und Untersuchungen von Wissenschaftlem auf diesem Gebiet, insbesondere der Biomechanik. Die dabei verwendeten Messmittel reichen von Hochfrequenzkameras über aufwändige Messstraßen und Messhufschuhen bis hin zu elektomyographischen Messgeräten in Verbindung mit Speziallaufbändern.

Bei allen bislang bekannten und angewandten Messmethoden besteht jedoch das Problem, dass sich kein Verfahren als absolut zuverlässig und praxistauglich erwiesen hat. Zudem sind die meisten Messverfahren mit hohem technischen Aufwand und meist nur unter Laborbedingungen möglich.

Eine exakte und unverfälschte Aufzeichnung und Auswertung des Bewegungsablaufes und / oder der Belastungsverteilung an den Hufen, insbesondere bei Pferden, setzt eine natürliche und gewohnte Umgebung des Tieres voraus. D.h. im Umkehrschluss, daß jede Ablenkung oder Veränderung der normalen Umgebungsbedingung direkt oder indirekt das Messergebnis unabhängig von der Meßmethode verfälscht.

Zu diesem Effekt kommt auch noch das Problem, welche Art von Messdaten eine aussagekräftige Relevanz besitzt. Neben den optischen Messverfahren werden bislang die bei der Fußung auftretenden Kräfte einerseits auf der Bodenseite, z. B. mittels Kraftmessplatten oder Messstraßen, und andererseits an der Hufunterseite gemessen, wie dies z. B. gemäß DE-A 195 46 022 in Form von Hufschuhen mit Messsensoren oder gemäß DE-A 197 07 413 in direkt unter dem Huf aufgeklebten Messsensoren erfolgt. Hierbei können auch Schwingungssensoren an den Beinen bzw. an den Hufen angebracht und die Messdaten über der Zeit aufgetragen werden.

Diese bekannten Kraftmeßverfahren können aber nur Segmente der real existenten Kräfte im lebenden Organismus, insbesondere im Huf, erfassen. Die für eine präzise Beurteilung notwendige komplexe Kräftemessung bzw. Kräfteverteilungsmessung, speziell im Huf, ist bislang nicht möglich.

Für die derzeitigen wissenschaftlichen Erkenntnisse wurden u. a. Untersuchungen an eingefrorenen und wieder aufgetauten Tierbeinen durchgeführt oder durch operativ implantierte Messsonden ermittelt.

Der Erfindung liegt die Aufgabe zugrunde, das Verfahren der gattungsgemäßen Art zur Beseitigung der beschriebenen Nachteile derart auszugestalten, daß hierdurch eindeutige und objektive Meßdaten, z.B. über die Kraft oder die Dehnung, erhalten werden, die sowohl für eine Bewegungsanalyse, insbesondere Lahmheitsdiagnostik, als auch über die Druckkraftverhältnisse im einzelnen Huf, z.B. Huf- oder Beinfehlstellung, Aufschluß geben; außerdem soll das zu schaffende Verfahren einfach zu bedienen sein, keine aufwändigen Laboraufbauten erfordern und das zu untersuchende Tier nicht in seinem natürlichen Bewegungsverhalten beeinflussen.

Die Merkmale des zur Lösung dieser Aufgabe geschaffenen Verfahrens gemäß der Erfindung ergeben sich aus Anspruch 1. Vorteilhafte Ausgestaltungen hiervon sind in den weiteren Ansprüchen beschrieben.

Grundsätzlich ist festzuhalten, dass sich die Komplexität des Körpers und des gesamten Bewegungsapparates auch im Aufbau des Hufes und seiner Funktionsweise als "Hufmechanismus" wiederspiegeln. Dabei werden die zwischen dem Körper und dem Boden wirkenden Kräfte von den Beinen, insbesondere aber auch vom Huf, übertragen. Diese mehrdimensionalen Kräfte werden überwiegend vom Tragrand des Hufes aufgenommen und über die Lederhaut auf die Knochen weitergeleitet. Zusätzlich können auch, je nach Bodenbeschaffenheit, über den Hufsohlenbereich Kräfte weitergegeben werden.

Mit dem erfindungsgemäßen Verfahren ist es nun möglich, einzelne oder mehrere dieser Kräfte an einem oder mehreren Hufen gleichzeitig oder nacheinander zu erfassen, als elektrisch auswertbare Datensätze aufzuzeichnen und in Bezug zueinander zu setzen. Die Messung kann dabei sowohl im Stand als auch in allen Gangarten des Tieres erfolgen. Außerdem ist es für die Messung unerheblich, ob das Tier bzw. der Huf einen Beschlag trägt oder nicht.

Die Grundlage für die Erfindung bildet die Tatsache, dass eine in einem Gegenstand existente Dehnung oder Stauchung über eine definierte Messlänge auf eine Messeinrichtung parallel so umgeleitet wird, dass die ursprüngliche Dehnung nicht nachteilig beeinflusst oder gar verfälscht wird. Durch die Übertragung der Dehnung auf die Messeinrichtung kann eine direkt proportionale Aussage über die im Gegenstand auftretenden Kräfte getroffen werden.

Die Erfindung wird im Folgenden anhand der Zeichnung näher erläutert. Es zeigen:
- Fig. 1: eine schematische Darstellung der Messeinrichtung an einem Equidenbein und

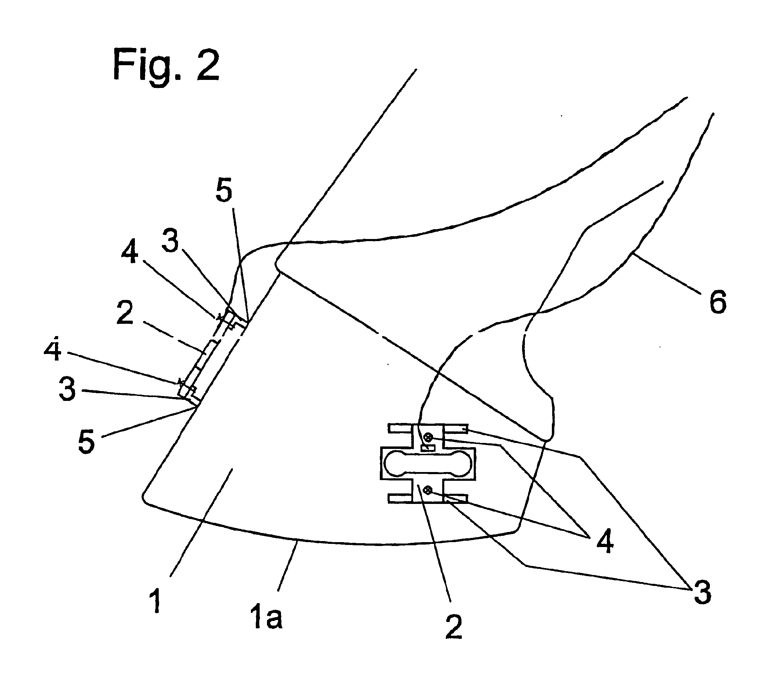

- Fig. 2: schematisch die Messeinrichtung in Drauf- und Seitenansicht auf dem Equidenhuf angebracht.

Für die Umsetzung der Dehnungsübertragung vom Hufhorn auf ein Messelement 2 werden zwei Kraftübertragungsstege 3 , vorzugsweise aus Aluminium, mit einem definierten Abstand auf eine ausgewählte Stelle auf dem Horn des Hufes 1 kraftschlüssig 5 fixiert. Die Fixierung erfolgt durch eine Klebeverbindung. Vorteilhaft für diese Klebeverbindung 5 ist ein schnellaushärtender Zweikomponentenkleber. Um diesen definierten Abstand zu erreichen, werden die beiden Kraftübertragungsstege 3 mit Hilfe einer Schablone in die entsprechende Position gebracht und während des Klebevorgangs gehalten. Nach dem Aushärten der Klebeverbindung 5 wird die Schablone entfernt und das Messelement 2 an ihrer Stelle eingesetzt. Für die ebenfalls kraftschlüssige Verbindung zwischen Messelement 2 und den Kraftübertragungsstegen 3 werden Schrauben 4 verwendet.

Die Dreiteilung der Messeinrichtung 2 und 3 ist zum einen für eine verspannungsarme Anbringung zweckmäßig und ermöglicht außerdem das Wiederverwenden des Messelementes 2, da es sich um eine lösbare Fügeverbindung 4 zwischen Kraftübertragungsstegen 3 und Messelement 2 handelt.

Auch das Ablösen der Kraftübertragungsstege 3 vom Huf 1 nach Beendigung der Messung verursacht keinerlei Schädigungen oder Verletzungen des Horns.

Die geometrische Auslegung und das verwendete Material des Messelementes 2, vorzugsweise Aluminium, sowie die definierte Länge der Messstrecke legen einen Proportionalitätsfaktor fest. Dieser Faktor stellt die Beziehung zwischen der tatsächlichen Dehnung im Horn und der Dehnung im Messelement 2 dar. Die Dehnung im Messelement 2 wird mit Dehnungsmessstreifen erfasst und in ein elektrisches Signal umgewandelt. Dieses Signal gibt, unter Einbeziehung des Proportionalitätsfaktors, Aufschluss über die real im Huf 1 auftretende Dehnung bzw. Kraft.

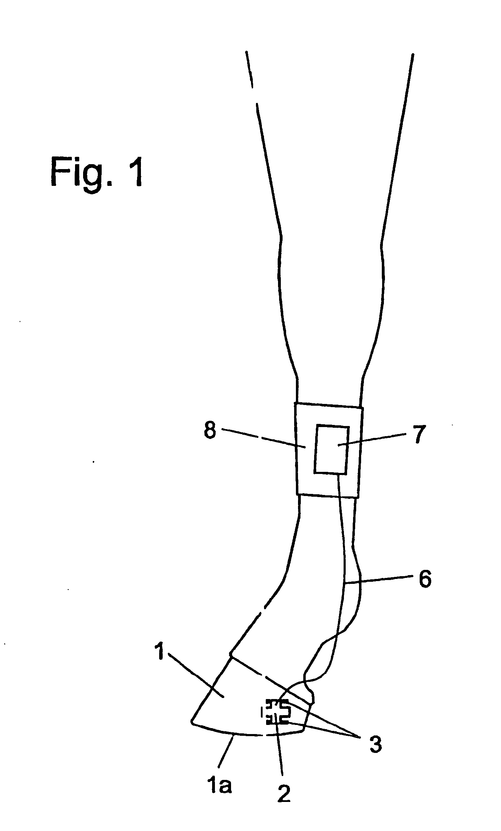

Das Messsignal des Messelementes 2 wird zu einem A/D-Wandler übertragen, und zwar über einen Transmitterverstärker 7, der vorzugsweise direkt am Messelement 2 selbst oder an einem Bauchgurt oder am Röhrbein des Tieres mit beispielsweise einer Gamasche 8 (siehe Fig.1), fixiert ist und die Messdaten per Kabel 6 vom Messelement 2 erhält. Der A/D-Wandler moduliert das Signal in eine digitale Datenform. Das Signal kann dabei in analoger oder digitaler Form, d.h. vor oder nach dem A/D-Wandler, telemetrisch vom bewegten Tier auf die ortsfeste Auswertestation übertragen werden. Diese digitalen Messdaten können von einem PC sowohl weiterverarbeitet als auch gespeichert werden.

Sinnvollerweise erfolgt die telemetrische Datenübermittlung per Funkübertragung, da so das Tier nicht in seiner Bewegungsfreiheit eingeschränkt wird und die Messungen in allen Gangarten durchgeführt werden können.

Die Auswertung der Messdaten erfolgt nach bereits praktizierten und gängigen Bearbeitungsmethoden. Die Art der Auswertung und die Auswahl der Stelle, auf der die Messvorrichtung fixiert wird, ist dabei abhängig von der beabsichtigten Gesamtaussage, z.B. als Bewegungsanalyse, zur Lahmheitsdiagnostik oder zur Ermittlung der Druckverteilung an einem einzelnen Huf oder im Vergleich von mehreren Hufen zueinander.

Trägt man beispielsweise die Messdaten der vertikalen Kräfte von jedem der vier Hufe mit vier an der äquivalenten Stelle am Huf platzierten Sensoren über der Zeit auf, kann eine Aussage über das Gangmuster und eventuelle Abweichungen (Lahmheit des Tieres) sichtbar gemacht werden.

Aber auch durch Vergleichsmessungen an einem Huf (z. B. Innenseite gegen Außenseite des Hufes) können Rückschlüsse auf die Belastung bzw. Belastungsverteilung, im Stand und / oder in allen verschiedenen Gangarten, gezogen werden (z. B. einseitige Abnutzung eines Hufes oder Hufeisens). Durch eine solche Vergleichsmessung kann präventiv (z. B. durch Korrektur der Hufstellung unter Verwendung von Keilen zwischen Huf und Hufeisen) gehandelt werden.

Sind die Messelemente entsprechend kalibriert und die Signale der Sensoren beim Entlasten des einzelnen Beines bzw. Hufes auf "Null" gesetzt, kann zusätzlich auch eine Aussage über das Gewicht bzw. die Gewichtsverteilung des Equiden gemacht werden.

Die Messgenauigkeit des Verfahrens hängt vom technisch betriebenen Aufwand ab, d. h. von der mechanischen und applikationstechnischen Fertigung des Messelementes und von der Verwendung der Materialien. Zudem muss sichergestellt sein, dass die kraftschlüssige Verbindung der eingesetzten Sensor-Elemente gewährleistet wird.

## Patentansprüche

1. Verfahren zur Ermittlung der Kraftverhältnisse an bzw. in Hufen (1), insbesondere von Equiden, wobei wenigstens eine Messeinrichtung an einer ausgewählten Stelle, ausgerichtet nach dem zu bestimmenden Kraftvektor, auf der Hufoberfläche angebracht ist,
**dadurch gekennzeichnet,**
**dass** die an der Oberfläche bzw. im Inneren eines Hufes (1) auftretenden Dehnungen und / oder Stauchungen von einer Messeinrichtung (2, 3) erfasst und in ein elektrisches Signal umgewandelt werden, wobei die Messeinrichtung (2, 3) dreiteilig ausgebildet ist und aus einem Sensorelement (2) sowie zwei Kraftübertragungsstegen (3) besteht, so dass der Unterschied zwischen den Elastizitätsmodulen des Hufhornes und des Sensorwerkstoffes ausgeglichen wird.

2. Verfahren nach Anspruch 1, **dadurch gekennzeichnet, dass** das Sensorelement (2) und die Kraftübertragungsstege (3) aus Aluminium oder Kupfer-Beryllium bestehen.

3. Verfahren nach Anspruch 1 oder 2, **dadurch gekennzeichnet, dass** durch die Messeinrichtung, insbesondere durch das Sensorelement (2), der Verformungskraft am bzw. im Huf (1) sowenig Kraft bzw. Dehnung entzogen wird, dass das Messergebnis nicht nachteilig beeinflusst und/oder verfälscht wird.

4. Verfahren nach einem der vorhergehenden Ansprüche, **dadurch gekennzeichnet, dass** die auf das Sensorelement (2) übertragene Dehnung in ein elektrisches Signal umgewandelt wird, vorzugsweise durch den Einsatz von Dehnungsmessstreifen.

5. Verfahren nach einem der vorhergehenden Ansprüche, **dadurch gekennzeichnet, dass** sowohl bei statischer als auch bei dynamischer Messung ein permanentes Messsignal anliegt.

6. Verfahren nach einem der vorhergehenden Ansprüche, **dadurch gekennzeichnet, dass** die Messeinrichtung an den Kraftübertragungsstellen (5) mit dem Hufhorn eine kraftschlüssige Fügeverbindung aufweist, vorzugsweise eine Klebeverbindung.

7. Verfahren nach Anspruch 6, **dadurch gekennzeichnet, dass** bei der Erstellung der Fügeverbindung das Sensorelement (2) durch eine Schablone ersetzt wird, um einen definierten Abstand der Kontaktflächen für die Krafteinleitung zu gewährleisten.

8. Verfahren nach einem der vorhergehenden Ansprüche, **dadurch gekennzeichnet, dass** das Sensorelement (2) mit den beiden Kraftübertragungsstegen (3) durch eine kraftschlüssige Verbindung, vorzugsweise Verschraubung (4), gekoppelt wird.

## Claims

1. Method of sensing the relationship of forces effecting on or in hoofs (1) of especially horses with at least one measuring means attached to the surface of the hoof at a selected point thereof, related to the factor force to be defined, **characterized in that** the extensions and/or compressions on the surface or inside of the hoof (1), respectively are realized by a measuring device (2, 3) and transferred into an electrical signal, wherein the measuring device is configured of three parts, namely of one sensor element (2) and two force transmitting bars (3) in order to equalize the difference between the elasticity modules of the horn of the hoof and the materials of the sensor.

2. Method according to claim 1, **characterized in that** the sensor element (2) and force transmitting bars (3) are comprised of a medium of copper-beryllium.

3. Method according to claim 1 or 2, **characterized in that** by the measuring device, especially by the sensor element (2), such small amount of force or extension, respectively is diminished from the deforming force on or in the hoof (1), respectively that the result of the measuring is not adversely influenced and/or falsified.

4. Method according to one of the preceding claims, **characterized in that** the extension transmitted from the sensor element (2) is transferred into an electrical signal preferably by using extensometer strips.

5. Method according to one of the preceding claims, **characterized in that** a permanent measuring signal is effecting either on measuring statically or on measuring dynamically.

6. Method according to one of the preceding claims, **characterized in that** the measuring device is provided at the force transmitting area (5) of the hoof surface with a force transmitting fitting connection, particularly an adhesive connection.

7. Method according to claim 6, **characterized in that** on preparing the fitting connection the sensor element (2) is replaced by a pattern in order to secure a defined distance of the contact surfaces for entering the force.

8. Method according to one of the preceding claims, **characterized in that** the sensor element (2) is connected to both of the force transmitting bars (3) by a force transmitting connection, preferably screwing (4).

## Revendications

1. Procédé pour connaître les rapports des forces sur ou dans des sabots d'animaux (1), en particulier d'équidés, dans lequel on monte sur la surface du sabot au moins un dispositif de mesure à un emplacement choisi, orienté selon le vecteur de force à déterminer,
**caractérisé en ce que**
les allongements et/ou les tassements apparaissant à la surface ou à l'intérieur d'un sabot (1) sont détectés par un dispositif de mesure (2, 3) et convertis en un signal électrique, le dispositif de mesure (2, 3) étant constitué de trois pièces que sont un élément détecteur (2) ainsi que deux barrettes de transmission de force (3), de sorte que la différence entre les modules d'élasticité de la corne du sabot et du matériau du détecteur est compensée.

2. Procédé selon la revendication 1, **caractérisé en ce que** l'élément détecteur (2) et les barrettes de transmission de force (3) sont constitués en aluminium ou en un alliage cuivre-béryllium.

3. Procédé selon la revendication 1 ou 2, **caractérisé en ce que** le dispositif de mesure, en particulier l'élément détecteur (2) prélève à la force de déformation sur ou dans le sabot (1) une force ou un allongement si faible que le résultat de mesure n'est pas influencé de façon négative et/ou falsifié.

4. Procédé selon l'une des revendications précédentes, **caractérisé en ce que** l'allongement transmis à l'élément détecteur (2) est converti en un signal électrique, de préférence par l'utilisation de jauges de contrainte.

5. Procédé selon l'une des revendications précédentes, **caractérisé en ce qu'**un signal de mesure permanent s'applique aussi bien lors d'une mesure statique que lors d'une mesure dynamique.

6. Procédé selon l'une des revendications précédentes, **caractérisé en ce que** le dispositif de mesure présente une liaison assemblée par coopération de formes, de préférence une liaison collée, avec la corne du sabot aux emplacements de transmission de force (5).

7. Procédé selon la revendication 6, **caractérisé en ce que** lors de l'établissement d'une liaison assemblée, l'élément détecteur (2) est remplacé par un gabarit pour assurer une distance définie des surfaces de contact pour l'application de la force.

8. Procédé selon l'une des revendications précédentes, **caractérisé en ce que** l'élément détecteur (2) est couplé aux deux barrettes de transmission de forces (3) par une liaison en coopération de forces, de préférence par vissage (4).
